# EUROPEAN PATENT SPECIFICATION

(11) **EP 3 218 661 B1**
(45) Date of publication and mention of the grant of the patent: **12.02.2020**
(21) Application number: 15800982.9
(22) Date of filing: 12.11.2015
(51) Int. Cl.: F26B 5/06, F26B 25/00, F26B 25/18

(54) **FREEZE DRYING OF NESTED VIALS**
GEFRIERTROCKNUNG VON VERSCHACHTELTEN PHIOLEN
LYOPHILISATION EN FLACONS PLACÉS DANS DES ALVÉOLES

(30) Priority: 14.11.2014 LU 92597
(43) Date of publication of application: 20.09.2017
(73) Proprietor: Project Pharmaceutics GmbH, 82152 Martinsried (DE)
(72) Inventor: SCHUETZ, Andreas, 82152 Martinsried (DE); HELLERBRAND, Klaus, 82152 Martinsried (DE)
(74) Representative: Schiweck Weinzierl Koch Patentanwälte Partnerschaft mbB
(86) International application number: PCT/IB2015/058742
(87) International publication number: WO 2016/075647

(56) References cited:
- WO-A1-2013/135826
- WO-A1-2013/147759
- WO-A2-2013/164422
- DE-A1- 2 235 483
- US-A- 4 325 193

## Description

### BACKGROUND

Lyophilization (or freeze-drying), the removal of the majority of the water in a sample under conditions of low temperature and vacuum, is a widely used technique in the pharmaceutical, biotechnological and food industry. Lyophilization is typically carried out in a freeze-dryer including a freeze drying chamber -which functions as a vacuum vessel- and shelves arranged in vertical stack in the chamber for holding the product or products to be freeze dried. The freeze dryer shelves are cooled and heated during the freeze drying cycle. Freeze-drying is split into three process stages, freezing, primary drying at lower temperatures and under vacuum wherein most of the solvent (water) is removed by sublimation, and secondary drying at ambient or higher temperatures to minimize the final unbound water content.

Lyophilization is used to dry and preserve various products such as food, pharmaceuticals, diagnostics, blood fractions, microorganisms or proteins. Since lyophilization greatly reduces weight, it makes products more convenient for storage, processing and transport. Moreover, removing water suppresses biological growth and chemical reactions and therefore increases the shelf-life of products. Compared to other drying techniques lyophilization has several advantages. Since the product is not exposed to high temperatures, lyophilization is particularly suitable for thermolabile substances. Furthermore lyophilization can be used for drying a product (e.g. food) while maintaining its basic shape. Another positive aspect is that freeze-dried products can be rapidly rehydrated due to their porous structure and large specific surface.

The ultimate objective of the freeze-drying process is to maintain product quality which is consistent not only within the batch but also from batch to batch. Freeze-drying is an expensive and time consuming process and hence is generally the focus in industry for process optimization. The prior art commonly strives after minimizing thermal resistance between the shelves and the bottom of the product containers in order to enable maximal heat transfer by conductivity from the shelf to the product for sublimation.

Hence, the containers (e.g., vials) are commonly placed directly on the shelves for processing. Alternatively, containers can be placed on a metal tray which is then transferred to the shelf. E.g., US 5,084,040 (West Co.) discloses lyophilization of specifically formed lyophilization vials in a metal tray which is envisaged to have a metal bottom and sides in order to maximize heat transfer from the shelves to the vials. The vials have been designed to have a substantially flat bottom in order to maximize heat transfer. US 5,084,040 also expresses a clear preference of trays with slide-out bottoms, in order to ensure direct contact between vials and shelf, thereby maximizing heat exchange. Also review articles from 2012, such as Hibler and Gieseler (2012), J Pharmaceutical Sciences, Vol. 101, No. 11, pp. 4025-4031 emphasize that the heat transfer between vial and shelf is of utmost importance for which reason vials and nests are designed such that the vial is in direct contact with the shelf. A most recent development that reflects direct contact between vials and shelf is the so-called adaptiQ™ system from Schott AG. The nest design of SCHOTT's adaptiQ™ system aligns vials, particularly the vial bottom, in one layer such that they can be lyophilized in the nest since it is ensured that the vials are through their bottom in direct contact with the shelf. The same is also true for WO2013/16442 (Schott AG) which emphasizes that vial bottoms shall be in direct contact with the shelves during lyophilization.

However, despite the efforts that have been undertaken in optimizing lyophilization so far, deviations in product quality are still experienced commonly due to an uneven heat distribution within the product container resulting in non-optimal lyophilization products with structural defects predominantly at the bottom region of the lyophilisate. To avoid overheating, shelf temperatures need to be controlled at relatively low temperature of usually below 0°C accompanied by correspondingly long process times. For example, in the method according to WO2013/147759 (Baxter Intl.), primary drying is performed at very low temperatures (between --70 °C and --25 °C) for a considerable time (> 64 hours). It is therefore the object of the present inventors to provide means and methods to satisfy the needs and demands set out in the prior art.

### SUMMARY

Much to their surprise and contrary to the prevailing opinion in the art which is also manifested in review articles and even text books, the present inventors observed that vials do not have to be in direct contact with the shelf of a lyophilization device in order to achieve an essentially homogenous/defect-free lyophilization product or composition. Specifically, the present inventors observed that a lyophilization product is obtainable by the means and methods of the present invention which has essentially no structural defects predominantly at the bottom region of the lyophilisate. This is achieved when the vial is not in direct contact with the shelf of a lyophilization device which is contrary to the traditional techniques and opinion in the prior art which constantly teaches that vials, in particular the bottom of the vial, has to be in direct contact with the shelf of a lyophilization device in the lyophilization process. In addition, the present inventors observed that lyophilization, in particular primary and/or secondary drying, is advantageously carried out at shelf temperatures from about +10°C to about +80°C, more preferably at shelf temperatures from about +20°C to about +60°C when there is no direct contact between a vial and the shelf of a lyophilization device. This finding is novel and was unexpected as it is contrary to the common lyophilization protocols that are known in the art (e.g. WO2013/147759) and which involve considerably lower primary drying temperatures below 0°C resulting in prolonged processing times. The methods and devices of the invention therefore allow for markedly faster and, hence, more efficient lyophilization that, in addition, preferably yield essentially homogenous and defect-free products.

Accordingly, the present invention provides a method according to claim 1. Preferred embodiments are recited in the dependent claims.

In one embodiment, said device is separated from the shelf of said lyophilization device by a spacer, thereby said vial comprising said composition is not in direct contact with the shelf of a lyophilization device.

In one embodiment, said method provides an essentially homogenous/defect-free lyophilized composition.

Preferably, desorption of the solvent is carried out at shelf temperatures from about +20°C to about +60°C.

In one embodiment, the device comprises an insulating material.

In one embodiment, the device provides insulation between the shelf and the vials by holding the vials above the shelf at a certain distance without any solid material between shelf and vial bottom. This may be achieved by holders.

In one embodiment, the insulating material is at least 1 mm high, however it can also have a height below 1 mm, such as 500 µm, 400 µm, 300 µm, 200 µm, 100 µm, 50 µm or 10 µm.

In one embodiment, said device is a nest array.

In a further aspect, an embodiment not forming part of the invention provides a device formed to house a plurality of vials preferably positioned adjacent to each other in a predetermined distance, wherein at least one vial of said plurality of vials is suitable to comprise a composition to be lyophilized, and
(i) wherein said device is separated from the shelf of a lyophilization device by a spacer; or
(ii) wherein vials adjacent to said vial suitable to comprise a composition to be lyophilized are separated from the shelf of a lyophilization device by a spacer, or
(iii) wherein vials are separated from the shelf in that they are positionedor suspended in a device, e.g., by holders or racks such that their bottom is not in direct contact with the bottom of the device; or
(iv) wherein vials are separated from the shelf in that they are positioned in a device such that their bottom is not in direct contact with the shelf, if the device in which the vials are positioned may not have a bottom; or
(v) wherein vials adjacent to said vial suitable to comprise a composition to be lyophilized protrude beyond said vial suitable to comprise said composition.
For (iii), (iv) and (v) the vials, so to say, hang in the air or in any other gas commonly used in a lyophilization device as described herein.

In yet a further aspect, use of a spacer in aiding in the preparation of an essentially defect-free lyophilized composition is envisaged, wherein said spacer is positioned between a device formed to accommodate a plurality of vials positioned adjacent to each other in a predetermined distance, wherein at least one vial of said plurality of vials is suitable to comprise a composition to be lyophilized, and a shelf of a lyophilization device. Yet, such s spacer may also be positioned between (a) vial(s) and the shelf.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range. It includes, however, also the concrete number, e.g., about 20 includes 20.

The term "less than" or "greater than" includes the concrete number. For example, less than 20 means less than or equal to. Similarly, more than or greater than means more than or equal to, or greater than or equal to, respectively.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of' excludes any element, step, or ingredient not specified in the claim element. When used herein, "consisting essentially of" does not exclude materials or steps that do not materially affect the basic and novel characteristics of the claim.

In each instance herein any of the terms "comprising", "consisting essentially of' and "consisting of' may be replaced with either of the other two terms.

It should be understood that this invention is not limited to the particular methodology, protocols, material, reagents, and substances, etc., described herein and as such can vary. The terminology used herein is for the purpose of describing particular embodiments only, and is not intended to limit the scope of the present invention, which is defined solely by the claims.

### DESCRIPTION OF THE FIGURES

**Figure 1****.** Schematic, exemplary illustration of vial positions in conventional freeze-drying (variant 1, left), nested freeze-drying (variant 2, middle) and nested freeze-drying with spacers (variant 3, right).
**Figure 2****:** Macroscopic appearance of variant 1 and 2 in comparison.

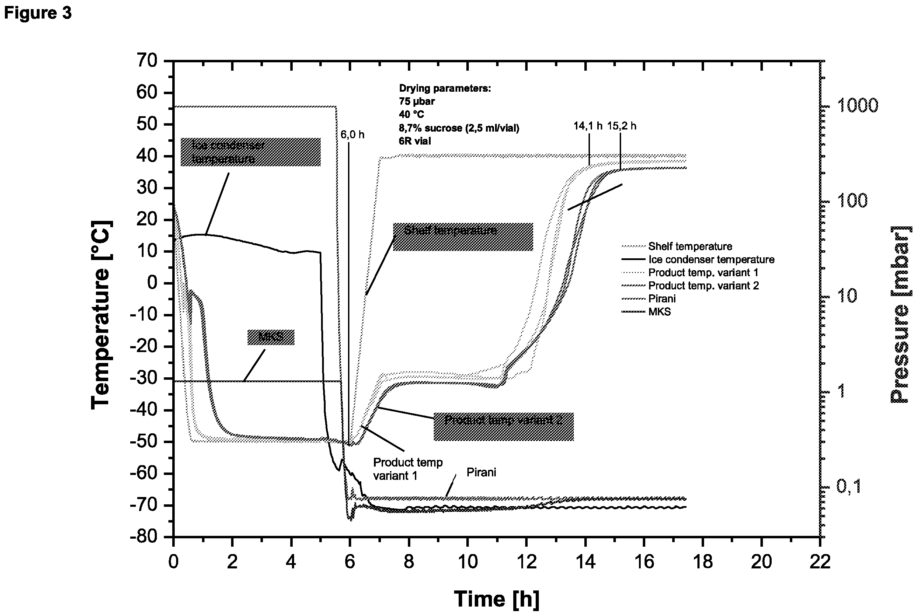

**Figure 3****:** Print out freeze-dryer experiment 1.
**Figure 4****:** Macroscopic appearance of variant 1, 2 and 3 in comparison.
**Figure 5****:** Print out freeze-dryer experiment 2.

### DETAILED DESCRIPTION

### Lyophilization

It is a traditional prejudice in the prior art that successful lyophilization requires maximum heat transfer between the shelves in a lyophilization chamber and the vials holding the products to be lyophilized in order to allow quick and defect-free freezing and sublimation. Therefore, the vials are generally placed directly on the shelves during lyophilization processing. The present inventors have however found that essentially defect-free lyophilisates can be obtained when direct contact between vials and shelf is avoided and the lyophilization vials are positioned adjacent to each other (i.e., "nested"): In fact, avoiding the direct shelf contact of the vials prevents local collapse or melting even when high shelf temperatures such as between +10°C to about +80°C, more specifically between +20°C to about +60°C are used to realize short lyophilization process times.

The present inventors have developed a novel lyophilization set-up that is combinable with any lyophilization protocol and advantageously yields an essentially homogenous/defect-free lyophilization product, particularly essentially free of structural defects at the bottom region of the lyophilisates, in particular at shelf-temperatures as described herein. Therefore, vials comprising the composition to be lyophilized are placed in a device which prevents the vials from being in direct contact with the shelf. This is in clear contradiction to conventional lyophilization set-ups known in the prior art.

In a first aspect, the present invention relates to a method for lyophilizing a composition comprising the features of claim 1. The preferred positioning of a plurality of vials is sometimes also referred to herein as "nested". Accordingly, a preferred device is a so-called nest.

The term "vial" when used herein may be replaced by the term "vessel", thus both terms are interchangeable. A vial may have every size and volume. A vial may have a flat bottom or an inwardly or partially inwardly curved bottom. Accordingly, for the latter vial only the margin of the bottom is in contact with a surface, i.e., a vial may be in a bulge-like contact with a shelf. All vials that are known and/or used in the market may be used in the methods of the present invention.

### Lyophilization protocol

Importantly, the inventive method is generally combinable with any lyophilization protocol. The term "lyophilization protocol" is used herein to describe the entirety of steps and parameters (such as temperature, pressure etc.) of a lyophilization process.

Common lyophilization protocols are based on the removal of solvent(s) such as water by sublimation and desorption and usually comprise the following three steps:
(1) Freezing (crystallization of solvent(s) at atmospheric pressure)
(2) Primary drying (sublimation of solidified solvent(s) under a vacuum)
(3) Secondary drying (desorption of non-crystallized adsorbed solvent residues)

As used herein, "shelf temperature" is temperature at which the lyophilizer is set. "Sublimation" is the transition from the solid state to a gas phase with no intermediate liquid phase which occurs during primary drying. After the composition is completely re-frozen, the pressure of the freeze dryer is reduced, and heat is applied to initiate sublimation of the ice or of the solvent crystal. "Desorption" occurs during secondary drying. It involves the removal of the excess moisture (e.g. bound water/solvent) by increasing the temperature of the product and reducing the partial pressure of water (solvent) vapor in the container. The term "thawing" or/and "melting" are used interchangeably to refer to the passage from a solid state to a liquid state. The thawing or the melting can be complete indicating a complete passage from the solid to the liquid phase or partial indicating the coexistence of a solid frozen and a liquid phase.

"Freezing" is the step in which the composition to be lyophilized is brought from a liquid state to a solid state. During the freezing step, ice/ solvent crystals form throughout the composition.

Usually, freezing is carried out at normal (atmospheric) pressure (about 1 bar) and aims to cool the composition until it is completely frozen, such that as much water as possible of (and/or other solvents) has passed from the liquid into the solid state. As freezing progresses, the solute phase becomes highly concentrated and is termed the "freeze concentrate." By the end of freezing, the freeze concentrate usually contains only about 20% of water (w/w), or less than 1% of total water in the solution before ice formation. The freezing step (1) may for instance be performed at a shelf temperature of -10 °C or less, -20 °C or less, -30 °C or less, -40 °C or less, -45 °C or less, -50°C or less, -55°C or less, -60°C or less, or -65°C or less.

Primary drying, or ice sublimation, begins whenever the chamber pressure is reduced and the shelf temperature is raised to supply the heat removed by ice sublimation. During primary drying, the chamber pressure is well below the vapor pressure of ice, and ice is transferred from the product to the condenser by sublimation and crystallization onto the cold coils/plates in the condenser. Primary drying is typically performed at reduced atmospheric pressure. The pressure is preferably set below 1 bar. E.g., the pressure during primary drying can be set to 5,0x10-3 bar or less, such as 1,0x10-3 bar or less, 1,0x10-4 bar or less, 5,0x10-5 bar or less. Primary drying is typically conducted for a time sufficient to ensure that the frozen solvent (e.g. water or another solvent), is removed from the frozen composition. One skilled in the art understands that the primary drying time varies in dependence of different parameters. The duration of the primary drying may depend on the fill volume, surface area of the cake, concentration etc. For example, primary drying may be conducted for 1 hour or more, such as 10, 20, 30, 40, 50 hours. However, it is envisaged that due to the high shelf temperatures applied in the methods of the invention, primary drying can be performed for a considerably shorter time than in the methods known in the art.

Secondary drying is the stage where water is desorbed from the freeze concentrate, usually at elevated temperature and low pressure. The pressure, preferably, is the same pressure of the primary drying step or a lower pressure as applied during the primary step. The secondary drying step is conducted for a time sufficient to reduce or remove the residual moisture level in the lyophilized product. The secondary drying step can be performed for a total time of at least 1 hour or at least 5 hours or more.

Notably, the lyophilization protocol as described herein allows for primary and/or secondary drying at particularly high shelf temperatures, such as between +10°C and +80°C, in particular between at +20°C and +60°C. This is advantageous in that it reduces overall processing time. In accordance with the foregoing, the primary drying step (2) and/or the secondary drying step may be performed at a shelf temperature of +10°C or more, such as +20°C or more, + 30°C or more, +40°C or more, +50°C or more, +60°C or more, +70°C or more, +80°C or more, +90°C or more, up to about +100°C.

Thus, the methods of the invention are envisaged to involve lyophilization comprising the following steps: (1) Freezing, (2) Primary drying ( at shelf temperatures of between +20°C and +60°C, as described above) and (3) Secondary drying (for instance at shelf temperatures of between +10°C and +80°C, in particular between at +20°C and +60°C, as described above).

E.g., lyophilization can be carried out as described in WO2013135826**.** Briefly, the atmosphere surrounding the vials is cooled to a temperature set at least below the eutectic melting temperature or the lowest eutectic melting temperature of the composition to be lyophilized. Then, the pressure of the surrounding atmosphere is reduced to below the pressure of the internal air inside the vials and the shelf temperature is raised.

However, it is to be understood that any lyophilization protocol is conceivable for use in accordance with the method of the present invention. That is, shelf temperatures for lyophilization will vary. In some embodiments, primary drying and/or secondary drying is carried out at shelf temperatures from about +20°C to about +60°C.

The person skilled in the art will be readily able to select a suitable lyophilization protocol depending on the composition to be lyophilized, the lyophilization equipment and/or the desired product characteristics.

### Vials

The term "vial" as used herein generally refers to a container suitable to hold the composition to be lyophilized during lyophilization and/or transport and storage. In general, the vials for use in accordance with the present invention include all vials that are preferably impermeable, stable to sterilization and chemically inert. The vials are further preferably structurally stable enough to withstand the operating temperatures, pressures etc. during freeze-drying. The vial can be a one-chamber vial, syringe, bag or ampoule. Preferably, the vial is a one-chamber vial, optionally comprising a stopper. The vials can be flexible or rigid. The vials can have any suitable form that allows them to be placed in a device, e.g. they can be tubular or molded. Suitable vials include vials made from glass (amber or clear), plastic or engineering polymers.

The term "plastic" includes, without limitation, polyester (PES), polyethylene terephthalate (PET), polyethylene (PE), High-density polyethylene (HDPE), polyvinyl chloride (PVC), polyvinylidene chloride (PVDC), medium-density polyethylene (MDPE), low-density polyethylene (LDPE), polypropylene (PP), polystyrene (PS), High impact polystyrene (HIPS), polyamides (PA) (Nylons), acrylonitrile butadiene styrene (ABS), polyethylene/acrylonitrile butadiene styrene (PE/ABS), polycarbonate (PC), polycarbonate/acrylonitrile butadiene styrene (PC/ABS), Polybutylene terephthalate (PBT), Polyoxymethylene (POM), SPS, thermoplastic elastomers (TPE), Polyphthalamide (PPA), Poly(p-phenylene sulfide (PPS), Polyether ether ketone (PEEK), Polyetherketone (PEK), PAI, Polyphenylsulfone (PPSU), PSU, Polycarbonate (PC), PC/PET, PCEm, Poly(methyl methacrylate) (PMMA), Styreneacrylonitrile resin (SAN), PS-HI, or combinations (co-polymers) thereof.

The term "engineering polymer" as used herein includes, e.g., cyclic olefin copolymer (COC).

It is envisaged that the vial comprising the composition to be lyophilized is comprised in a plurality of vials which are positioned adjacent to each other at a predetermined distance in a device as described herein. This configuration is also described as "nested lyophilization" herein. Without wishing to be bound by a specific theory, it is thought that a nested arrangement of the vials enables homogenous energy input, thereby favoring local shrinkage or melting in particular in the bottom region, resulting in uniform lyophilization of the composition to be lyophilized. The term "adjacent" encompasses the vials being positioned left, right, in front of and/or behind each other. When the vials are positioned in a device comprising preformed depressions as described elsewhere herein, the term "adjacent" preferably means that the vials are positioned such that no depressions are left empty between two vials. The term encompasses that the vials may or may not contact each other. The term "plurality of vials" as used herein means at least 2, 3, 4, 5, 6, 7, 8, 9, 10 or more vials. It is in particular envisaged that at least 1, 2, 3 or 4 vials are positioned adjacent to the vial comprising the composition to be lyophilized. However, the number of adjacent vials may be even higher depending on the geometric shape of the vials and the device. The vials adjacent to the vial comprising the composition to be lyophilized may comprise said composition, too.

### Device/"Nest"

Any device can be used in accordance with the present disclosure as long as it allows positioning the vials adjacent to each other in a predetermined distance (i.e., "nested") and is suitable to prevent the vial(s) comprising the composition to be lyophilized from being in direct contact with the shelf during lyophilization. Such a device is also termed "nest" herein.

Without wishing to be bound by specific theory, it is contemplated that avoiding direct contact between the vial and the shelf favors a uniform energy input avoiding local collapse or melting, thereby enabling defect-free lyophilization. The uniform energy input allows the use of high shelf temperatures for rapid drying, e.g. shelf temperatures between +20°C and +60°C during primary and/or secondary drying.

In one embodiment, the device comprises a side section and an upper section, and preformed depressions protruding from the upper section which are capable of housing the lyophilization vials, preferably in a predetermined distance. When the device is placed within the lyophilization chamber, the bottom section of the preformed depressions contact the shelf, preventing the vials from coming in direct contact with the shelf. In some embodiments of the invention, the device is a premolded plastic tray in which the vials are typically supplied.

In another embodiment, the device is a tray comprising a bottom section, side sections and vial holder to position the vials adjacent to each other in a predetermined distance. Said vial holder can, e.g., be an inset comprising voids in a predetermined distance for receiving the vials. The bottom section of the tray will prevent the vials from coming into direct contact with the shelf. That is, in some embodiments of the invention, the device itself will prevent the vials from coming into direct contact with the shelf.

In another embodiment, the device is a tray comprising side sections and vial holder to position the vials adjacent to each other in a predetermined distance. Said vial holder can, e.g., be an inset comprising voids in a predetermined distance for receiving the vials. The device may be designed in way that no bottom is present with the vials fixed in a certain position above the shelf without contacting the shelf.

The device can be made from a variety of materials, including plastic, metal or engineering polymers. In general, the same materials as described in the context of the vials are conceivable. It is thus envisaged that the device may be rigid, keeping the vials in a predetermined distance from each other and from the shelf. Alternatively, the device could also be flexible, allowing for the vials to move relatively to each other for a certain degree. In one embodiment, the device is a nest array.

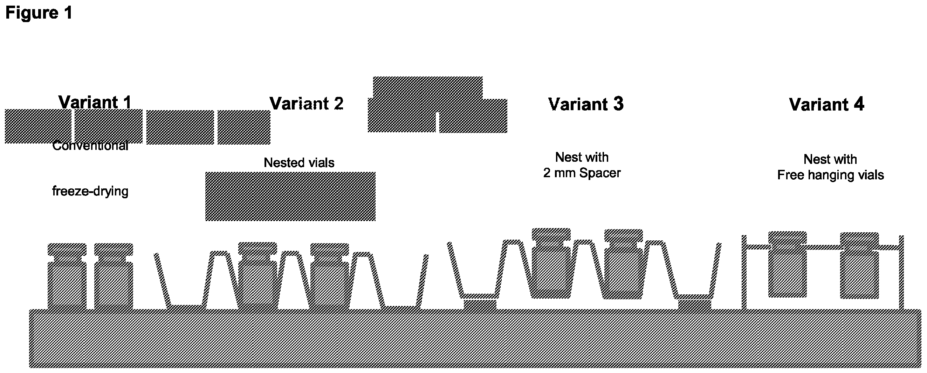

It is contemplated that the bottom section of the device used in accordance with the methods of the invention prevents the vials from coming into direct contact with the shelf. In some embodiment, additional spacers can be used to ensure that the vials do not come into direct contact with the shelf (see Fig. 1 for exemplary embodiments). It is therefore also envisaged that, in some embodiments, the device is separated from the shelf by a spacer, such that the vial comprising the composition to be lyophilized is not in direct contact with the shelf of a lyophilization device. The spacer may be positioned between the vial comprising the composition to be lyophilized and the shelf or the spacer may be positioned between the adjacent vials and the shelf such that the vial comprising the composition to be lyophilized is separated from the shelf by an empty space (see Fig. 1 for an exemplary embodiment). The same effect can be achieved if the device itself is formed such that the vials comprising the composition to be lyophilized are separated from the shelf by an empty space, e.g. by providing a device having one or more separating elements which prevent the vials from coming into direct contact with the shelf.

The expression "between the vial and the shelf" in this context means the spacer being positioned between the part of the device holding the vial and the shelf. When the spacer separates the vial comprising the composition to be lyophilized from the shelf, it is conceivable to position the spacer between the device and the vials, e.g. within the bottom part of the preformed depressions of the device. The spacers can, additionally or alternatively, be placed between the device and the shelf, e.g. between the bottom parts of the preformed depressions and the shelf.

In accordance with the foregoing, the expression whereupon the vial comprising said composition to be lyophilized is "not in direct contact" with the shelf of a lyophilization device therefore encompasses all embodiments wherein the bottom part of the device separates the vial from the shelf, and wherein optionally an additional spacer and/or empty space separates the vial from the shelf. The term "not in direct contact" when used herein also includes that a vial, particularly the bottom of a vial is not in direct contact with the shelf of a lyophilization device. For example, the bottom of a vial is not in direct contact with the shelf of a lyophilization device, if a vial may not have a flat bottom, but an inwardly curved bottom. Accordingly, only the margin of the bottom of the vial may be in contact with the shelf, while almost the entire bottom is not. Such a vial is encompassed by the scope of the present invention. Another example for not being in direct contact with the shelf is a vial that comprises an insulating material as described herein. Such a vial is encompassed by the scope of the present invention. Just another example is a vial that is fixed, for example, by a holder of a device, e.g. a tray or rack or nest such that its bottom is not in direct contact with the shelf. Accordingly, any gas commonly used in a lyophilization device may be in between the space between the bottom and the shelf. Such a device is encompassed by the scope of the present invention. A just further example is a vial separated from the shelf by a spacer, wherein the spacer is preferably made of an insulating material. Such a spacer and device comprising one or more of such spacers is encompassed by the scope of the present invention.

The present disclosure therefore also encompasses a device formed to house a plurality of vials positioned adjacent to each other in a predetermined distance, wherein at least one vial of said plurality of vials is suitable to comprise a composition to be lyophilized, and
(i) wherein said device is separated from the shelf of a lyophilization device by a spacer,
(ii) wherein vials adjacent to said vial suitable to comprise a composition to be lyophilized are separated from the shelf of a lyophilization device by a spacer, or
(iii) wherein vials adjacent to said vial suitable to comprise a composition to be lyophilized protrude beyond said vial suitable to comprise said composition.

Further, use of a spacer in aiding in the preparation of an essentially defect-free lyophilized composition is envisaged by the present disclosure, wherein
(i) said spacer is positioned between a device formed to accommodate a plurality of vials positioned adjacent to each other in a predetermined distance, wherein at least one vial of said plurality of vials is suitable to comprise a composition to be lyophilized, and a shelf of a lyophilization device, or
(ii) wherein said spacer is positioned between at least two vials adjacent to a vial suitable to comprise a composition to be lyophilized, and a shelf of a lyophilization device.

The device and/or the spacers preferably comprise an insulating material. The term "insulating" as used herein means "having thermal insulation capacity", i.e. reducing heat transfer/thermal conductivity (the transfer of thermal energy between objects of differing temperature) between objects in thermal contact.

Likewise, a vial may comprise an insulating material. The insulating material may be temporarily or permanently adhered or fixed to a vial. For example, it may be sprayed on a vial in a thin layer such as a SiOₓ layer. The vial may comprise or be coated with an insulating material in a manner as described herein below, e.g. fully coated, partially coated, in particular only the bottom of the vial and/or any other part.

Thermal conductivity is the quantity of heat transmitted through a unit thickness in a direction normal to a surface of unit area, due to a unit temperature gradient under steady state conditions. It is expressed in watts per meter kelvin (W/(mK)). The term "insulating" in all its grammatical forms when used in the context of the present invention can in particular mean "having a thermal conductivity of less than about 200 k, more preferably less than about 150, less than about 100, less than about 50, less than about 25, less than about 10, less than about 5 k when measured at 25°C by modulated differential scanning calorimetry (DSC) as described in Marcus SM and Blaine RL. Thermochim Act. 1994. 243 (2): 231-239. In some particular embodiments, the device comprises an insulating material having a thermal conductivity of less than about 2, 1.75, 1.5, 1.25, 1.0, 0.75, 0.50, 0.25, 0.15, 0.10, 0.005 W/(mK) when measured at 25°C by modulated differential scanning calorimetry (DSC) as described in Marcus SM and Blaine RL. (loc cit).

Insulating materials for use in accordance with the present invention include, without limitation, plastics, e.g. as described elsewhere herein, expanded polystyrene (EPS), silica aerogel, polyurethane, polyethylene, polypropylene, acrylnitril-butadien-styrol (ABS), polyisocyanurate (ISO), urea-formaldehyde, polystyrene, fiberglass, icynene, cellulose, wood, glass, perlite, air, SiOₓ, empty space, vacuum or combinations thereof.

A further insulating material for use in accordance with the present invention also include all kinds of gases used in the lyophilization chamber at the pressure used during lyophilization, such as nitrogen, argon, xenon.

Several embodiments are envisaged by the present disclosure. In one embodiment, the device itself comprises an insulating material. E.g., the whole device can be made from an insulating material, such as plastic. It is also conceivable that only the bottom part of the device which comes into contact with the shelf is made from an insulating material. One or more of the side parts, the top part and/or the vial holder can also be made from an insulating material. If the device comprises preformed depressions, only the depressions or only the bottom part of the depressions which come into contact with the shelf can be made from an insulating material. In another embodiment the device has no bottom and the vials are fixed in a certain position above the shelf without contacting the shelf. It is also envisaged that the device comprises insulating spacers.

The insulating material is preferably at least 1mm high. That is, the insulating material can be 1.5, 2.0, 2.5, 3.0, 3.5, 4.0, 4.5, 5.0, 5.5, 6.0, 6.5, 7.0, 7.5, 8.0, 8.5, 9.0, 9.5, 10.0 mm high or higher.

### Composition

In general, any composition can be lyophilized according to the method of the present invention. "Lyophilized composition" and "lyophilizate" or, in short, "composition" are to be understood as equivalent. The terms "lyophilizate" and "lyophilized composition" refer to the solid remaining after the lyophilization process. As used herein "lyophilizate" and "lyophilized composition" describe the product of a lyophilization.

The term "composition" includes pure substances, suspensions, gels, liquids, solutions, solids, or mixtures thereof. For example, the inventive method is suitable for lyophilization of compositions comprising pharmaceutically active ingredients, biopharmaceutical materials, foodstuffs, food additives, feed, chemical materials, vaccines, and may include products such as wound-care products, cosmetics, veterinary products and in vivo/in vitro diagnostics related products and the like. In some embodiments, the pharmaceutically active ingredient can be any active ingredient, including, but not limited to, proteins, nucleic acids, peptides, viruses, nucleic acids, small molecules and combinations thereof. Proteins can include, but are not limited to, glycoproteins, clotting factors, growth factors, cytokines, hormones, antibodies, and chimeric constructs. The term "protein" herein is intended to be broad and refers to individual or collective native human or other mammalian proteins; and/or homogenous or heterogeneous distribution of polypeptides arising from a single or multiple gene products; and/or fragments of proteins displaying a particular activity; and/or such proteins and/or active fragments thereof produced by recombinant techniques including transgenic technology.

Other illustrative materials useful in the present method include biological or biopharmaceutical material such as tissues, organs and multi-cellular structures. For certain biological and pharmaceutical applications, the material may be a solution or mixture that includes: a live or attenuated viruses; nucleic acids; monoclonal antibodies; polyclonal antibodies; biomolecules; non-peptide analogues; peptides, including polypeptides, peptide mimetics and modified peptides; proteins, including fusion and modified proteins; RNA, DNA and subclasses thereof; oligonucleotides; viral particles; and similar such materials or components thereof.

### Excipients

In some embodiments, a composition comprising one or more pharmaceutically active ingredients further comprises a pharmaceutical excipient such as a buffer, tonicity modifier, stabilizer, bulking agent, excipient or a combination thereof.

As used herein, "pharmaceutical excipient" includes any and all solvents, dispersion media, antioxidants, salts, bulking agents (sugars, sugar alcohols, amino acids) ,v surfactants, preservatives (e.g., methyl or propyl p-hydroxybenzoate, sorbic acid, antibacterial agents, antifungal agents), isotonic agents, solution retarding agents (e.g., paraffin), absorbents (e.g., kaolin clay, bentonite clay), drug stabilizers (e.g., sodium lauryl sulphate), gels, binders (e.g., syrup, acacia, gelatin, sorbitol, tragacanth, polyvinyl pyrrolidone, carboxy-methyl-cellulose, alginates), excipients (e.g., lactose, milk sugar, polyethylene glycol), disintegration agent (e.g., agar-agar, starch, lactose, calcium phosphate, calcium carbonate, alginic acid, sorbitol, glycine), wetting agents (e.g., cetyl alcohol, glycerol monostearate), , absorption accelerators- (e.g., quaternary ammonium salts), edible oils (e.g., almond oil, coconut oil, oily esters or propylene glycol), sweetening agents, flavoring agents, coloring agents, fillers, (e.g., starch, lactose, sucrose, glucose, mannitol), carriers for inhalation (e.g., hydrocarbon propellants), buffering agents, or such like materials and combinations thereof, as would be known to one of ordinary skill in the art.

Pharmaceutical excipients used in lyophilization are known in the art and e.g. described in Baheti A. et al: Excipients used in lyophilization of small molecules. JJournal of Excipients and Food Chemicals; 1: 41-54, 2010 and in "Remington's Pharmaceutical Sciences" by E.W. Martin or in "Handbook of Pharmaceutical Excipients" by R. C. Rowe et al.

### Solvent

The composition to be lyophilized typically comprises a solvent. Water is the most commonly used solvent. Beside water also other solvents or solvent mixtures with water are used for lyophilization as long as those solvents or solvent mixtures can be frozen or freeze dried under technically feasible conditions.

The composition to be lyophilized can comprise an organic solvent. Exemplary organic solvents for use in the method of the present invention include ethanol, solketal (2,2-Dimethyl-1,3-dioxolan-4-yl)methanol), methanol, 1-propanol, 2-propanol, 1-butanol, 2-butanol, tert-butanol, glacial acetic acid, anisole, benzylalcohol, pentanol (all iso forms), DMSO, N-methyl-2-pyrrolidon, acetone, methylethylketone, ethylacetate, isobutylacetate, isopropylacetate, methylacetat, 3-methyl-1-butanol, propylacetate, ethylether, tert-butylmethylether, or a combination thereof, with and without water.

### Product

Under appropriate conditions lyophilization removes solvents, while leaving the basic structure of a material intact or resulting into a powder. Lyophilization products therefore typically emerge as coherent entities having a spongy porous structure. The solid content remaining after the freeze drying process is usually called 'cake'. Preferably, the methods of the present invention result in the generation of an essentially homogenous, defect-free lyophilized composition, particularly in a lyophilized composition that is essentially free of structural defects in the bottom region of a lyophilisate. "Bottom region" meaning that part of a lyophilisate that is located at or was or is in contact with the bottom of a vial. A "lyophilisate" is solid and also includes a lyophilization cake or lyophilization intermediate.

"Homogenous" or "defect-free" means that the product is preferably uniform in structure, color and/or texture and preferably does not show signs of structural defects, shrinkage, clumping and/or collapse. Whether a product possesses the desired properties is assessable by its macroscopic and microscopic appearance. Preferably, the product is comprises porous, interstitial openings and is readily soluble.

Ideally, the inventive method yields a dense white cake with fine, uniform structure, showing good physical strength and friability, is obtained. Alternatively, the present invention also envisages a fine, homogenous powder to be obtained.

Lyophilized products obtainable by any of the method of the invention include, but are not limited to pharmaceutical compositions, nutraceutical compositions, intermediates in the production of vaccines, pharmaceuticals or nutraceutical, food, feed, additives to food or feed. Lyophilized products obtainable with any of the method of the invention further include, compounds not for human or animal use like e.g. intermediates of chemical synthesis or ceramics.

### Examples

Vials with identical test solution composition (8.7 % sucrose, 2.5 ml/vial) were freeze-dried. Three different sample variants were tested: In a conventional variant vials were dried directly on the shelf. In a 2^{nd} variant, vials were dried in nests and in a further variant an additional distance of 2 mm between nest and shelf was arranged by using spacers beneath the nest.

### Materials and equipment

### Materials

### Used materials are listed in Table 1.

**Table 1: Materials**

| **Substances** | **Lot.** | **Manufacturer** | **Order no.** |
|---|---|---|---|
| Sucrose Ph. Eur | 051165320 | Merck | 466.1 |

| **Consumables** | | | |
|---|---|---|---|
| 6R Vials, FIOLAX glass clear | K42784453 205 | MGIas | 1.07653.9029 |
| 20 mm stopper; V9154; FM257/2 | 30320433 | Helvoet | |
| Polymer nest | n.a. | Nipro Glass Germany AG | n.a. |

### Equipment

**Freeze-Dryer:**

| | |
|---|---|
| • Manufacturer: | Hof Sonderanlagenbau (Lohra, Germany) |
| • 0.5 m² shelf area | |
| • 10 kg condenser capacity | |

**Camera**

| | |
|---|---|
| • Type: | EOS 550D |
| • Manufacturer: | Canon (Tokio, Japan) |
| | |
| • Lense: | Sigma 105mm 2,8 EX DG Macro |
| • Manufacturer: | Sigma (Kawasaki, Japan) |

**Analytical Balance**

| | |
|---|---|
| • Typ: | 1602 MP |
| • Manufacturer: | Sartorius (Göttingen, Germany) |

**Balance**

| | |
|---|---|
| • Type: | Kern 572 |
| • Manufacturer: | Kern (Balingen, Germany) |

**Pipettes**

| | |
|---|---|
| • Type: | Pipetman 1000 µL; EE 94639 |
| | Pipetman 5000 µL; EE 56692 |
| • Manufacturer: | Gilson (Middleton, USA) |
| | |
| • Type: | HandyStep electronic |
| • Manufacturer | Brand (Wertheim, Germany) |

### Example 1:

6R type glass vials were filled with 2.5 ml sucrose solution 8.7% (w/w). Two variants were prepared. For variant 1, vials were loaded directly on the shelf of the freeze-dryer as in conventional vial freeze-drying processes. Variant 2 vials were placed in a nest (Figure 1). Additional 2 mm spacers were used to additionally increase the distance to the shelf (Figure 1).

The vials were processed with a modern single step, high speed lyo cycle charted in Table 2. End of drying was determined by product temperature monitoring via thermo couples.

**Table 2: Freeze-drying parameters**

| **Step** | **Step duration** | **Total duration** | **Shelf temperature** | **Condenser temperature** | **Pressure** |
|---|---|---|---|---|---|
| | **[hh:mm]** | **[hh:mm]** | **[°C]** | **[°C]** | **[mbar]** |
| | | | | | |
| **Equilibration** | 00:05 | 0:05 | 20 | 0 | 1000 |
| **Freezing** | 00:30 | 0:35 | -50 | 0 | 1000 |
| **Freezing** | 05:00 | 5:35 | -50 | 0 | 1000 |
| **Evacuation** | 00:30 | 6:05 | -50 | -80 | 7,50E-02 |
| **Drying** | 01:00 | 7:05 | 40 | -80 | 7,50E-02 |
| **Drying** | 10:00 | 17:05 | 40 | -80 | 7,50E-02 |

### Macroscopic examination

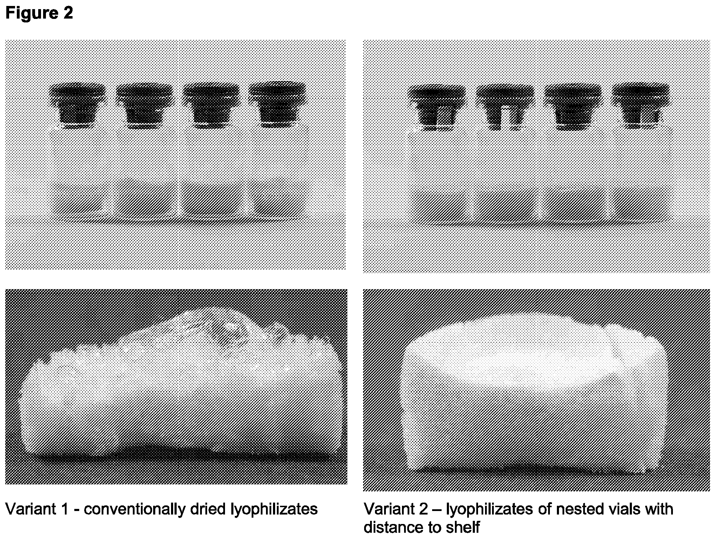

Figure 2 shows both lyophilizate variants. Lyo samples of variant 1 (vials directly positioned on the shelf) had clear defects with a cavernous structure indicating severe collapse. The cut lyophilizate showed bubble-like structures which is an indicator for melting. The second variants showed a homogeneous structure and no cavities in the lyo cakes.

### Lyo cycle records

Product temperature data shows a sharp increase of product temperature about 8-9 h after beginning of heating indicating end of sublimation. This is well in line with an approximation of MKS to Pirani read-out indicating that the chamber is free of water vapour from this point in time on. Sublimation period of the nested variants took about 1 h longer in comparison to the conventional variant.

### Example 2:

6R vials were filled with 2.5 ml sucrose solution 8.7% (w/w). In this experiment results of experiment 1 should be confirmed. Additionally a nest was positioned directly on the freeze-dryer's shelf to evaluate the influence of a larger distance between nest and shelf on drying speed and product quality (Figure 1).

The vials were processed with the lyo cycle described in experiment 1 (table 2). End of drying was determined by product temperature monitoring via thermo couples.

### Macroscopic examination

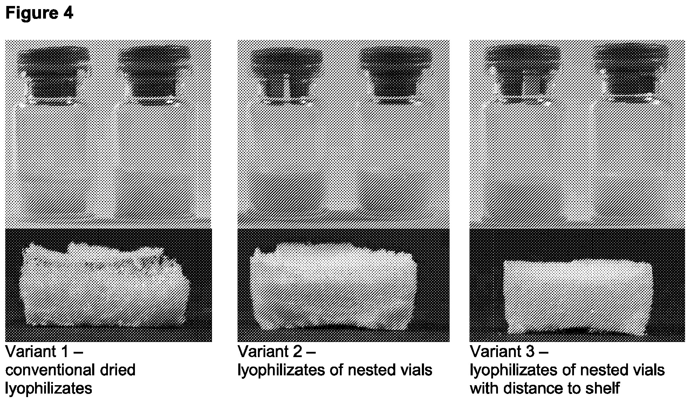

Figure 4 shows the lyophilizate variants. As already shown in experiment 1, conventional variant (variant 1) dried directly on the shelf showed collapse: Meltings and cavities in the lyophilizate structure. Variants 2 and 3, dried in the nests resulted in comparable lyophilizates without defects.

### Lyo cycle records

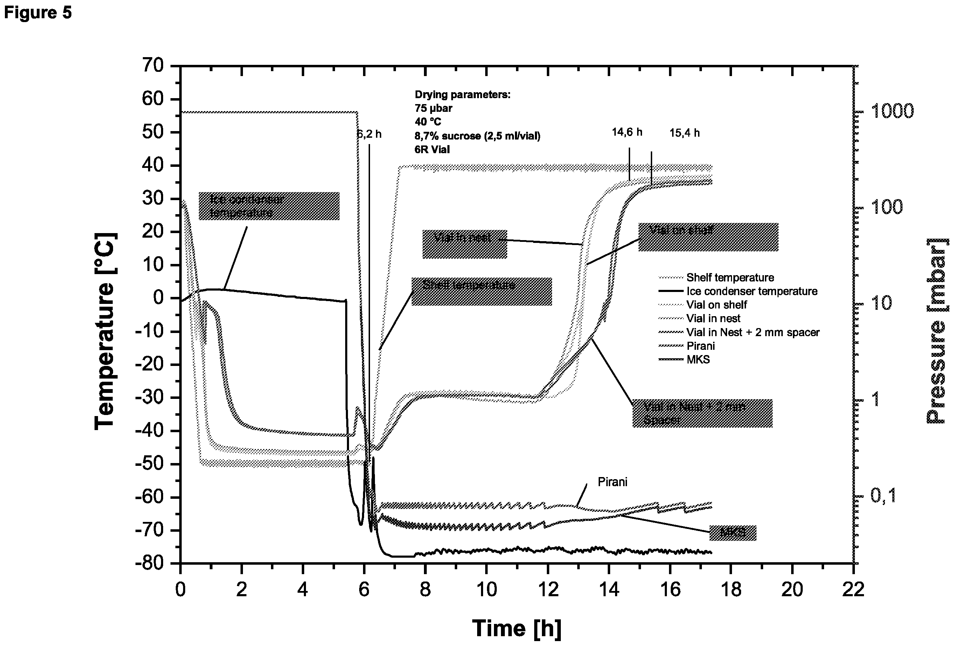

End of primary drying of all variants was comparable (Figure 5). Product temperature data shows a sharp increase of product temperature about 8-9 h after beginning of heating indicating end of sublimation. This is well in line with an approximation of MKS to Pirani read-out indicating that the chamber is free of water vapour from this point in time on. Sublimation period of the nested variants with spacer took about 1 h longer in comparison to the conventional variant and to the variant in nests. In this case the nested vials without additional spacers showed a comparable drying time to the vials dried directly on the shelf.

### Conclusion

The Lyo print outs of the examples 1 and 2 showed that freeze-drying at +40 °C shelf temperature and a pressure of 75 µbar resulted in comparable drying times. However, cake appearance differed significantly with clear defects in case of variants processed conventionally and homogenous, defect free structure in case of nested vials.

In contrast to the conventional drying, the insulated drying in nests allows to manufacture essentially defect-free lyophilizates specifically when using high speed lyophilization cycles with only marginally prolongation of drying time caused by the insulation.

## Claims

1. A method for lyophilizing a composition comprising lyophilizing a composition comprised in a vial, said vial being comprised in a plurality of vials housed by a device, wherein said vial comprising said composition to be lyophilized is not in direct contact with the shelf of a lyophilization device, the method being **characterized in that** primary drying is carried out at shelf temperatures from about +20°C to about +60°C.

2. The method for lyophilizing a composition of claim 1, wherein said plurality of vials is positioned adjacent to each other.

3. The method for lyophilizing a composition of claim 2, wherein said plurality of vials is positioned adjacent to each other, in a predetermined distance.

4. The method of claim 1, 2 or 3, wherein said device is separated from the shelf of said lyophilization device by a spacer, thereby said vial comprising said composition is not in direct contact with the shelf of a lyophilization device.

5. The method of claim 1, 2 or 3, wherein said vials are not in direct contact with the bottom of said device.

6. The method of claim 1, 2, 3 or 5, wherein said vials are positioned or suspended in the device such that they are not in direct contact with the bottom of said device or with the shelf if the device has no bottom.

7. The method of claim 1, 2 or 3, wherein said vials have an inwardly curved bottom.

8. The method of any one of the preceding claims, wherein primary and secondary drying is carried out at shelf temperatures from about +20°C to about +60°C.

9. The method of any one of the preceding claims, comprising
(a) providing at least one device formed to accommodate a plurality of vials positioned adjacent to each other in a predetermined distance, wherein at least one vial of said plurality of vials comprises a composition to be lyophilized;
(b) introducing said at least one device into a lyophilization device; and
(c) lyophilizing the composition to be lyophilized.

10. The method of any one of the preceding claims, wherein the device or vial comprises an insulating material.

11. The method of any one of the preceding claims, wherein said device is a nest array.

## Patentansprüche

1. Verfahren zum Lyophilisieren einer Zusammensetzung, umfassend Lyophilisieren einer Zusammensetzung, die in einem Probengefäß enthalten ist, wobei das Probengefäß in einer Mehrzahl von Probengefäßen enthalten ist, die in einer Vorrichtung aufgenommen sind, wobei das Probengefäß, das die zu lyophilisierende Zusammensetzung aufweist, nicht in direktem Kontakt mit dem Regal einer Lyophilisierungsvorrichtung steht, wobei das Verfahren **dadurch gekennzeichnet ist, dass** die Primärtrocknung bei Regaltemperaturen von etwa +20°C bis etwa +60°C durchgeführt wird.

2. Verfahren zum Lyophilisieren einer Zusammensetzung nach Anspruch 1, wobei die Mehrzahl von Probengefäßen benachbart zueinander angeordnet ist.

3. Verfahren zum Lyophilisieren einer Zusammensetzung nach Anspruch 2, wobei die Mehrzahl von Probengefäßen benachbart zueinander in einem vorbestimmten Abstand zueinander angeordnet ist.

4. Verfahren nach Anspruch 1, 2 oder 3, wobei die Vorrichtung durch einen Abstandshalter von dem Regal der Lyophilisierungsvorrichtung getrennt ist, wodurch das Probengefäß, das die Zusammensetzung aufweist, nicht in direktem Kontakt mit dem Regal einer Lyophilisationsvorrichtung steht.

5. Verfahren nach Anspruch 1, 2 oder 3, wobei die Probengefäße nicht in direktem Kontakt mit dem Boden der Vorrichtung stehen.

6. Verfahren nach Anspruch 1, 2, 3 oder 5, wobei die Probengefäße in der Vorrichtung so positioniert oder aufgehängt sind, dass sie nicht in direktem Kontakt mit dem Boden der Vorrichtung oder mit dem Regal stehen, wenn die Vorrichtung keinen Boden aufweist.

7. Verfahren nach Anspruch 1, 2 oder 3, wobei die Probengefäße einen nach innen gekrümmten Boden aufweisen.

8. Verfahren nach einem der vorstehenden Ansprüche, wobei die Primär- und Sekundärtrocknung bei Regaltemperaturen von etwa +20°C bis etwa +60°C durchgeführt wird.

9. Verfahren nach einem der vorstehenden Ansprüche, umfassend
(a) Bereitstellen mindestens einer Vorrichtung, die so ausgebildet ist, dass sie eine Mehrzahl von Probengefäßen aufnimmt, die in einem vorbestimmten Abstand nebeneinander angeordnet sind, wobei mindestens ein Probengefäß aus der Mehrzahl von Probengefäßen eine zu lyophilisierende Zusammensetzung aufweist;
(b) Einführen der mindestens einen Vorrichtung in eine Lyophilisationsvorrichtung; und
(c) Lyophilisieren der zu lyophilisierenden Zusammensetzung.

10. Verfahren nach einem der vorhergehenden Ansprüche, wobei die Vorrichtung oder das Probengefäß ein Isoliermaterial aufweist.

11. Verfahren nach einem der vorhergehenden Ansprüche, wobei die Vorrichtung eine Schachtelanordnung ist.

## Revendications

1. Procédé de lyophilisation d'une composition comportant la lyophilisation d'une composition comprise dans un flacon, ledit flacon étant compris dans une pluralité de flacons logée dans un dispositif, ledit flacon comportant ladite composition à lyophiliser n'étant pas en contact direct avec l'étagère d'un dispositif de lyophilisation, le procédé étant **caractérisé par le fait qu'**un séchage primaire est effectué à des températures d'étagère d'environ +20°C à d'environ +60°C.

2. Procédé de lyophilisation d'une composition selon la revendication 1, ladite pluralité de flacons étant positionnés adjacents les uns aux autres.

3. Procédé de lyophilisation d'une composition selon la revendication 2, ladite pluralité de flacons étant positionnés adjacents les uns aux autres à une distance prédéterminée.

4. Procédé selon la revendication 1, 2 ou 3, ledit dispositif étant séparé de l'étagère dudit dispositif de lyophilisation par un espaceur, ainsi ledit flacon comportant ladite composition n'est pas en contact direct avec l'étagère d'un dispositif de lyophilisation.

5. Procédé selon la revendication 1, 2 ou 3, lesdits flacons n'étant pas en contact direct avec le fond dudit dispositif.

6. Procédé selon la revendication 1, 2, 3 ou 5, lesdits flacons étant positionnés ou suspendus dans le dispositif en sorte qu'ils ne sont pas en contact direct avec le fond dudit dispositif ou avec l'étagère, si le dispositif n'a pas de fond.

7. Procédé selon la revendication 1, 2 ou 3, lesdits flacons ayant un fond courbé vers l'intérieur.

8. Procédé selon l'une quelconque des revendications précédentes, le séchage primaire et secondaire étant effectué à des températures de l'étagère d'environ +20°C à d'environ +60°C.

9. Procédé selon l'une quelconque des revendications précédentes comportant
(a) fournir au moins un dispositif conçu pour recevoir une pluralité de flacons positionnés adjacents les uns aux autres à une distance prédéterminée, au moins un flacon de ladite pluralité de flacons comportant une composition à lyophiliser;
(b) introduire ledit au moins un dispositif dans un dispositif de lyophilisation; et
(c) lyophiliser la composition à lyophiliser.

10. Procédé selon l'une quelconque des revendications précédentes, le dispositif ou le flacon comportant un matériau isolant.

11. Procédé selon l'une quelconque des revendications précédentes, ledit dispositif étant un agencement en alvéoles.
